# EUROPEAN PATENT APPLICATION

(11) **EP 4 667 040 A1**
(43) Date of publication of application: **24.12.2025**
(21) Application number: 23922255.7
(22) Date of filing: 24.07.2023
(51) Int. Cl.: A61M 25/09, A61B 17/22

(54) **GUIDE WIRE SYSTEM, GUIDE WIRE VIBRATION CONTROL METHOD, AND COMPUTER-READABLE STORAGE MEDIUM**

(30) Priority: 16.02.2023 CN 202310128047
(71) Applicant: Shanghai Microport Rotapace Medtech Co., Ltd., Shanghai 201203 (CN)
(72) Inventor: CHEN, Zhixiong, Shanghai 201203 (CN); LIU, Peifeng, Shanghai 201203 (CN); YAO, Yingzhong, Shanghai 201203 (CN); YUE, Bin, Shanghai 201203 (CN); CHANG, Zhaohua, Shanghai 201203 (CN)
(74) Representative: Murgitroyd & Company
(86) International application number: PCT/CN2023/108833
(87) International publication number: WO 2024/169132

(57) **Abstract**

This application relates to a guidewire system, a guidewire vibration control method, and a computer-readable storage medium. The guidewire system includes a driving module (100) and a guidewire (200). The driving module (100) includes a coil assembly (110) and a magnetic field generator (120). The magnetic field generator (120) is configured to generate a magnetic field. When an alternating current is provided to the coil assembly (110), the coil assembly (110) is able to perform axial reciprocating motion in the magnetic field, thereby driving the guidewire (200) to generate axial vibration.

## Description

### RELATED APPLICATIONS

This application claims priority to Chinese Patent Application No. 202310128047.5, filed on February 16, 2023, entitled "Guidewire System, Guidewire Vibration Control Method, and Computer-Readable Storage Medium", the entire content of which is incorporated herein by reference.

### TECHNICAL FIELD

The present application relates to the technical field of medical devices, particular to a guidewire system, a guidewire vibration control method, and a computer-readable storage medium.

### BACKGROUND

Chronic Total Occlusion (CTO) of a coronary artery refers to 100% occlusion of the coronary artery lumen. This disease has a low success rate in Percutaneous Coronary Intervention (PCI) surgery and a relatively high incidence of complications. At present, the main clinical treatment for this disease is to recanalize the CTO lesion. Successful recanalization of the CTO lesion can relieve the patient's angina symptoms, improve left ventricular function, stabilize the electrical activity of the myocardium, and thereby enhance the patient's tolerance to future coronary events.

In conventional technologies, when the CTO intervention and recanalization treatment is carried out, generally, the operator delivers a thin and long guidewire to the vicinity of the diseased tissue with the help of imaging. Then, the guide wire penetrates the CTO tissue under a certain force to achieve recanalization. Devices such as piezoelectric transducers can be used to drive the end of the guidewire to vibrate, so as to facilitate the direct penetration of the CTO. The mechanism has the effect of a hand drill, which can effectively penetrate the CTO tissue without requiring a large penetration force and will not cause damage to normal tissues, improving the overall safety and stability of the system. However, the piezoelectric crystals of the piezoelectric transducer will wear out during use, resulting in a relatively short service life and high cost of the piezoelectric transducer.

### SUMMARY

According to the embodiments of the present application, a guidewire system, a guidewire vibration control method, and a computer-readable storage medium are provided.

A guidewire system is provided, which includes a guidewire and a driving module. The driving module includes a magnetic field generator configured to generate a magnetic field and a coil assembly. When an alternating current is provided to the coil assembly, the coil assembly is able to perform axial reciprocating motion in the magnetic field, thereby driving the guidewire to generate axial vibration.

In an embodiment, the coil assembly includes a coil and a coil frame. The coil is disposed on the coil frame, and the coil frame is connected to a tail end of the guidewire.

In an embodiment, an axial guide slot is provided on the coil frame, and a tail end of the magnetic field generator is positioned in the axial guide slot. This enables the coil frame to perform axial reciprocating motion along the magnetic field generator. The coil is wound around an outer wall of the coil frame.

In an embodiment, the coil assembly further includes a connecting rod, a head end of the connecting rod is connected to the tail end of the guidewire, and a tail end of the connecting rod is connected to the coil frame after passing through the magnetic field generator and the axial guide slot in sequence.

In an embodiment, the magnetic field generator includes a large diameter section and a small diameter section, the large diameter section and the small diameter section are connected in sequence along a direction from head ends to tail ends of their own. An outer diameter of the large diameter section is greater than that of the small diameter section. The outer diameter of the large diameter section is greater than a slot width of the axial guide slot. The coil frame is able to perform axial reciprocating motion along the small diameter section.

In an embodiment, the coil assembly further includes a magnetic yoke. The magnetic yoke is disposed on the coil frame and distributed around the coil.

In an embodiment, the coil assembly further includes a shock absorber, and the shock absorber is disposed between the coil frame and the magnetic field generator.

In an embodiment, the shock absorber is a spring.

In an embodiment, the driving module further includes a housing, both the coil assembly and the magnetic field generator are disposed inside the housing. A tail end of the guidewire enters the housing to connect with the coil assembly.

In an embodiment, outer diameter of a head end of the guidewire gradually decrease in a direction away from the magnetic field generator.

In an embodiment, a material of a head end of the guidewire is a thermosensitive material. Hardness of the thermosensitive material at a first preset temperature is greater than the hardness of the thermosensitive material at a second preset temperature. The first preset temperature is greater than the second preset temperature.

In an embodiment, a surface of a head end of the guidewire is covered with a developing layer.

In an embodiment, the guidewire system further includes a control module. The control module is configured to provide the alternating current to the coil assembly and adjust a frequency of the alternating current.

In an embodiment, an adjustment button is provided on the control module. The control module adjusts the frequency of the alternating current when it is detected that the adjustment button is pressed.

In an embodiment, a display unit is provided on the control module. The display unit is configured to display the frequency of the alternating current.

A guidewire vibration control method using the guidewire system according to any one of the above embodiments. The guidewire vibration control method includes:
generating the magnetic field by using the magnetic field generator of the guidewire system, and providing the alternating current to the coil assembly of the guidewire system, enabling the coil assembly to perform axial reciprocating motion in the magnetic field, thereby driving the guidewire of the guidewire system to generate axial vibration.

A computer-readable storage medium is provided. The computer-readable storage medium stores a computer program thereon. The computer program, when executed by a processor, implements the steps of the guidewire vibration control method according to the above guidewire vibration control method.

Details of one or more embodiments of the present application are set forth in the following drawings and description. Other features, objectives, and advantages of the present application will become apparent from the specification, drawings, and claims.

### BRIEF DESCRIPTION OF THE DRAWINGS

To better describe and explain the embodiments and/or examples of this application disclosed here, reference may be made to one or more drawings. Additional details or examples used to describe the drawings should not be considered as limiting the scope of the disclosed application, the currently described embodiments and/or examples, or the currently best schemes of this application.
FIG. 1 is a schematic perspective structural diagram of a guidewire system according to one or more embodiments.
FIG. 2 is a schematic structural diagram of a driving module after perspective processing according to one or more embodiments.
FIG. 3 is a brief structural diagram of a guidewire according to one or more embodiments.

### DETAILED DESCRIPTION OF THE EMBODIMENTS

The following description will clearly and completely describe the technical solutions in the embodiments of this application in conjunction with the accompanying drawings. Obviously, the described embodiments are only a part of the embodiments of this application, not all of them. Based on the embodiments in this application, all other embodiments obtained by those skilled in the art without inventive effort are within the protection scope of this application.

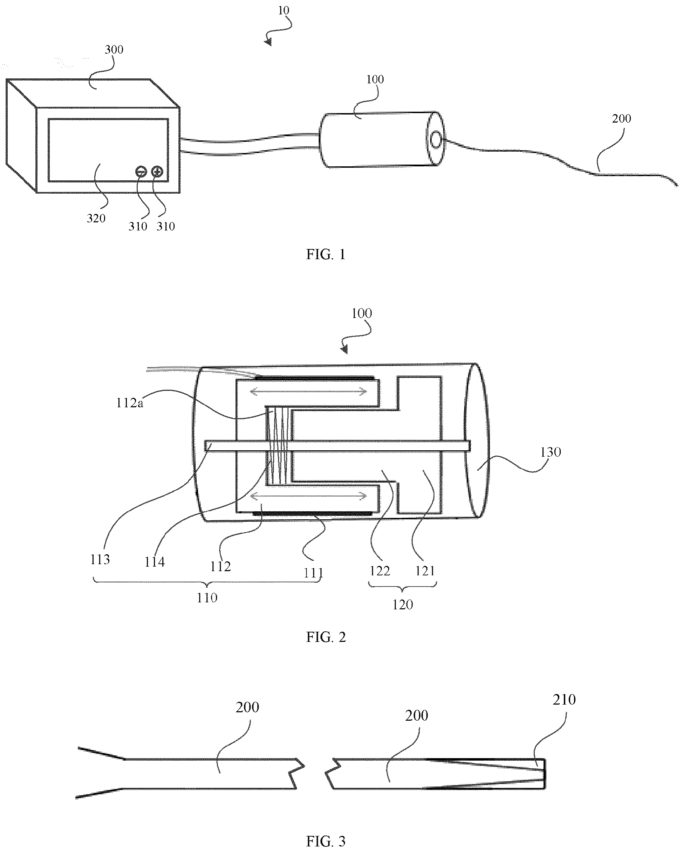

Referring to FIG. 1, FIG. 1 shows a guidewire system provided in an embodiment of this application. The guidewire system includes a connected driving module 100 and guidewire 200. Furthermore, the driving module 100 includes a coil assembly 110 and a magnetic field generator 120. When an alternating current passes through the coil assembly 110, the coil assembly 110 can perform axial reciprocating motion in the magnetic field generated by the magnetic field generator 120, thereby enabling the coil assembly 110 to drive the guidewire 200 to generate axial vibration.

In the guidewire system described above, the coil assembly 110 can perform axial reciprocating motion in the magnetic field generated by the magnetic generator 120 when provided with an alternating current, thereby enabling the coil assembly 110 to drive the guidewire 200 to generate axial vibration, making the head end of the guidewire 200 to produce an impact force on a calcified lesion tissue (such as CTO tissue). This impact force can cause fragmentation or atomization of calcification at the point of action on the lesion tissue, allowing for rapid penetration to the lesion tissue, improving effectiveness and efficiency for passing through hard lesions, and alleviating the problem of a long time for CTO treatment surgery. Moreover, the present application uses a means of electromagnetic moving coil to generate vibration of the guidewire 200, which does not have the problem of wear in piezoelectric crystals, thus extending the service life of the guidewire system and reducing costs.

Below is a description of each component of the guidewire system.

### (1) Driving Module 100

### (1.1) Coil Assembly 110

As shown in FIG. 2, in some embodiments of the present application, the coil assembly 110 can include a coil 111 and a coil frame 112. The coil 111 is disposed on the coil frame 112, and the coil frame 112 is connected to the tail end of the guidewire 200. The coil assembly 110 of this structure facilitates the fixation of the coil 111 and the guidewire 200. It should be noted that the double-headed arrow in FIG. 2 indicates the motion direction of the coil assembly 110.

Optionally, the coil 111 may be copper wires. When the alternating current is provided to the coil 111, an alternating magnetic field can be generated. The frequency of the alternating current can be adjusted within a range from 0kHz to 30kHz.

Optionally, the coil frame 112 can be made of an insulating material. For example, it can be made of high-frequency dielectric materials such as polytetrafluoroethylene or polystyrene to reduce losses.

As shown in FIG. 2, in some embodiments of the present application, an axial guide slot 112a can be provided on the coil frame 112, and the tail end of the magnetic field generator 120 is positioned in the axial guide slot 112a, allowing the coil frame 112 to perform axial reciprocating motion along the magnetic field generator 120. The coil 111 is wound around the outer wall of the coil frame 112. The axial guide slot 112a can guide the motion of the coil frame 112, enabling the coil frame 112 to perform axial reciprocating motion along the magnetic field generator 120, facilitating the axial vibration of the guidewire 200. In addition, by providing the axial guide slot 112a on the coil frame 112, the coil 111 can be wound around the outer wall of the coil frame 112, eliminating the need to dispose the coil 111 on the inner wall of the axial guide slot 112a, thus avoiding friction between the coil 111 and the magnetic field generator 120, and facilitating the electrical connection between the coil 111 and an external device. In some other embodiments, the axial guide slot can also be provided on the magnetic field generator 120, allowing the coil frame 112 to perform axial reciprocating motion along the axial guide slot. As an example, the coil 111 can be wound around the outer wall of the coil frame 112 using a manner of multi-layer winding.

It should be noted that the profile of the axial guide slot 112a matches the contour of the magnetic field generator 120. For example, if the magnetic field generator 120 is cylindrical, the axial guide slot 112a would be a circular slot with dimensions matching the dimensions of the magnetic field generator 120. Similarly, if the magnetic field generator 120 is a polygonal prism, the axial guide slot 112a would be a polygonal slot with dimensions matching the dimensions of the magnetic field generator 120. The slot width of the axial guide slot 112a can be slightly larger than the outer diameter of the magnetic field generator 120, which can reduce friction between the coil frame 112 and the magnetic field generator 120.

In some embodiments of the present application, as shown in FIG. 2, the coil assembly 110 further includes a connecting rod 113. An head end of the connecting rod 113 is connected to an tail end of the guidewire 200. An tail end of the connecting rod 113 is connected to the coil frame 112 after passing through the magnetic field generator 120 and the axial guide slot 112a in sequence. The connecting rod 113 facilitates the connection between the guidewire 200 and the coil frame 112.

It can be understood that a through-hole is provided on the magnetic field generator 120 for the connecting rod 113 to pass through. The diameter of the through-hole can be slightly larger than the outer diameter of the connecting rod 113, avoiding friction between the connecting rod 113 and the magnetic field generator 120 when the coil frame 112 performs axial reciprocating motion.

Optionally, the tail end of the connecting rod 113 can be connected to the coil frame 112 in a manner such as adhesive bonding or threaded connection. The material of the connecting rod 113 can be the same as or different from the material of the coil frame 112.

In some embodiments of the present application, the coil assembly 110 can further include a magnetic yoke (not shown in the FIGS). The magnetic yoke is disposed on the coil frame 112 and distributed around the coil 111. The magnetic yoke can reduce the amount of magnetic flux leakage in the magnetic circuit to provide a greater thrust to the guidewire 200 under the same alternating current, enabling the guidewire 200 to pass through the lesion tissue faster, and also minimizing the alternating current required to achieve the same vibration intensity in driving the guidewire 200.

Optionally, the magnetic yoke may be made of materials such as soft iron, A3 steel, or soft magnetic alloy. The magnetic yoke can be in the form of sheets and can be disposed on the coil frame 112 in a manner such as adhesive bonding, snap-fit, or fasteners (e.g., screws). The specific distribution of the magnetic yoke on the coil frame 112 can be determined according to specific situations. For example, the magnetic yoke can be distributed on both sides of the coil 111 along the axial direction.

As shown in FIG. 2, in some embodiments of the present application, the coil assembly 110 further includes a shock absorber 114 which is disposed between the coil frame 112 and the magnetic field generator 120. The shock absorber 114 can reduce the vibration of the driving module 100 and also lower the noise of the driving module 100.

Optionally, the shock absorber 114 may be a spring. The bottom of the axial guide slot 112a can be provided with a first limiting post (not shown in the FIG.s). The wall of the magnetic field generator close to the axial guide slot 112a can be provided with a second limiting post (not shown in the FIG.s). The head end of the shock absorber 114 can be sleeved over the second limiting post, and the tail end of the shock absorber 114 can be sleeved over the first limiting post. The limitation provided by the first and second limiting posts can prevent the shock absorber 114 from falling off.

### (1.2) Magnetic Field Generator 120

Given that the axial guide slot 112a is provided on the coil frame 112, as shown in FIG. 2, in some embodiments of the present application, the magnetic field generator 120 can include a large diameter section 121 and a small diameter section 122. The large diameter section 121 and the small diameter section 122 are connected in sequence along the direction from the head ends to the tail ends of their own. The outer diameter of the large diameter section 121 is greater than the outer diameter of the small diameter section 122, and the outer diameter of the large diameter section 121 is greater than the slot width of the axial guide slot 112a. The coil frame 112 can perform axial reciprocating motion along the small diameter section 122. The magnetic field generator 120 of this structure can limit the axial motion amplitude of the coil frame 112.

It should be noted that the outer diameter of the small diameter section 122 is less than or equal to the slot width of the axial guide slot 112a, and the axial length of the small diameter section 122 is greater than or equal to the slot depth of the axial guide slot 112a.

In some embodiments of the present application, the magnetic field generator 120 may be a magnet. It can be understood that when the coil frame 112 performs axial reciprocating motion, the magnetic field generator 120 remains stationary.

### (1.3) Housing 130

As shown in FIG. 2, in some embodiments of the present application, the driving module 100 further includes a housing 130. Both the coil assembly 110 and the magnetic field generator 120 are disposed inside the housing 130, and the tail end of the guidewire 200 enters the housing 130 to connect with the coil assembly 110. The housing 130 can be held by an operator, facilitating smooth operation during surgery.

Optionally, the length of the housing 130 may be within a range of 15cm~30cm (for example, 15cm, 20cm, 25cm, 30cm, etc.), and the diameter can be within a range of 10mm~30mm (for example, 10mm, 15mm, 20mm, 25mm, 30mm, etc.). Setting the dimensions of the housing 130 in this way makes it convenient for the operator to hold. The housing 130 may be an injection-molded part. This can reduce the weight of the driving module 100, making it even more convenient for the operator to hold. Regarding the shape of the housing 130, it can be cylindrical, polygonal prism-shaped, or ergonomically contoured. The embodiments of the present application do not impose specific restrictions, as long as it is convenient for the operator to hold.

Optionally, the magnetic field generator 120 can be fixed inside the housing 130 in a manner such as adhesive bonding or snap-fitting.

### (2) Guidewire 200

It can be understood that the guidewire 200 is used for insertion into the patient's blood vessels to allow the operator to use the guidewire system of the present application for recanalization to chronic total occlusions in the patient's coronary arteries. The main body of the guidewire 200 can be made from one or more metal materials or polymers, typically using materials with balanced flexibility and support, such as stainless steel, nickel-platinum alloy, nickel-titanium alloy, polyurethane, or polytetrafluoroethylene. It should be noted that the main body of the guidewire 200 refers to all parts of the guidewire 200 except for the head end thereof.

In some embodiments of the present application, the length of the guidewire 200 may be within a range of 1.5m~3.5m (for example, 1.5m, 2m, 2.5m, 3m, 3.5m, etc.), and the diameter of the guidewire 200 may be within a range of 0.1mm~0.5mm (for example, 0.1mm, 0.2mm, 0.3mm, 0.4mm, 0.5mm, etc.). The specific dimensions can be chosen according to the actual use requirements of application scenarios to meet sufficient operational length for insertion, pushability within blood vessels, and penetration performance of the head end of the guidewire 200.

As shown in FIG. 3, in some embodiments of the present application, the outer diameter of the tail end of the guidewire gradually decreases in the direction away from the magnetic field generator 120. This configuration of the tail end of the guidewire 200 facilitates the threaded connection of threaded component (such as screw) on the tail end of the guidewire 200 to threaded components (such as nuts) on the connecting rod 113.

As shown in FIG. 3, in some embodiments of the present application, the outer diameter of the head end of the guidewire 200 gradually decreases in the direction away from the magnetic field generator 120. Since the guidewire 200 needs to pass through tortuous blood vessels via accesses such as the radial artery or peripheral arteries, the entire guidewire 200 as a whole, especially the head end of the guidewire 200, needs to have good flexibility. Excessive hardness of the head end may prevent the head end from passing through bends or cause vascular damage during delivery. By configuring the outer diameter of the head end of the guidewire 200 in this way, the flexibility of the head end of the guidewire 200 can be improved.

Optionally, the length of the head end of the guidewire 200 can be within a range of 3cm~10cm (for example, 3cm, 4cm, 5cm, 6cm, 7cm, 8cm, 9cm, 10cm, etc.), and the minimum diameter of the head end of the guidewire 200 can be half the diameter of the main body of the guidewire 200. This ensures that the head end of the guidewire 200 can smoothly pass through the lesion tissue.

In some embodiments of the present application, the material of the head end of the guidewire 200 can be a thermosensitive material. The hardness of the thermosensitive material at a first preset temperature is greater than the hardness of the thermosensitive material at a second preset temperature, where the first preset temperature is greater than the second preset temperature. During the delivery of the guidewire 200 inside the body, when the temperature of the guidewire 200 is relatively low, the head end of the guidewire 200 presents better flexibility, facilitating delivery. During treatment, as the temperature of the guidewire 200 increases, the hardness of the head end of the guidewire 200 increases, facilitating a rapid passage of the head end of the guidewire 200 through lesions.

Optionally, the material of the head end of the guidewire 200 can be selected from at least one of thermosetting resin, polyurethane, or polytetrafluoroethylene.

As shown in FIG. 3, in some embodiments of the present application, the surface of the head end of the guidewire 200 can be covered with a developing layer 210. The developing layer 210 can meet the positioning requirements of the guidewire 200.

Optionally, the developing layer 210 can be made of metallic materials such as gold or platinum, and can be coated on the surface of the head end of the guidewire 200 by using electroplating.

It should be noted that after the surface of the head end of the guidewire 200 is covered with the developing layer 210, the outer diameter of the head end of the guidewire 200 can be the same as the outer diameter of the main body of the guidewire 200, facilitating the delivery of the guidewire 200.

### (3) Control Module 300

As shown in FIG. 1, in some embodiments of the present application, the guidewire system may further include a control module 300. The control module 300 is conFIG.d to provide alternating current to the coil assembly 110 and adjust the frequency of the alternating current. As an example, the control module 300 can be electrically connected to the coil 111 of the coil assembly 110 via wires. The control module 300 can adjust the frequency of the alternating current, allowing the coil 111 to be used without resonant frequency limitations. This enables adjustment of the vibration frequency of the guidewire 200 according to different surgical needs or different stages during surgery, improving the safety and effectiveness of the surgery.

It should be noted that the frequency of the output voltage of the control module 300 is consistent with the frequency of the output current (i.e., the alternating current provided to the coil assembly 110), and the vibration frequency of the guidewire 200 can be adjusted by adjusting the frequency of the output voltage. In some embodiments, the control module 300 can also be configured to adjust the amplitude, the duty cycle, and the duration of the output voltage, thereby changing the vibration mode of the guidewire 200, i.e., changing the amplitude, duty cycle, and duration of the vibration of the guidewire.

As shown in FIG. 1, in some embodiments of the present application, an adjustment button 310 is provided on the control module 300. The control module (300) adjusts the frequency of the alternating current when it is detected that the adjustment button 310 is pressed. The operator can set the frequency of the alternating current to a preset frequency by pressing the adjustment button 310, facilitating the surgery.

Optionally, the control module 300 includes a detecting unit, a processing unit, and a regulating unit. The detecting unit is configured to detect pressing on the adjustment button 310 and send a detection result to the processing unit. The processing unit is configured to send an instruction signal to the regulating unit when the detecting unit detects that the adjustment button 310 is pressed. The regulating unit is configured to adjust the frequency of the alternating current according to the instruction signal.

Optionally, the adjustment button 310 may be a mechanical button or a touch button. There may be two adjustment buttons 310, where one adjustment button 310 can be configured to decrease the frequency of the alternating current, and the other adjustment button 310 can be configured to increase the frequency of the alternating current. To distinguish between these two adjustment buttons 310, identifiers can be provided on the adjustment buttons 310. For example, a "+" symbol can be added to the adjustment button 310 configured to increase the frequency of the alternating current, and a "-" symbol can be added to the other adjustment button 310 configured to decrease the frequency of the alternating current. For sure, there can also be 10 adjustment buttons 310, representing the numbers "0", "1", "2", "3", "4", "5", "6", "7", "8", "9" respectively. The operator can set the specific frequency of the alternating current by pressing different adjustment buttons 310.

As shown in FIG. 1, in some embodiments of the present application, a display unit 320 is provided on the control module 300. The display unit 320 is configured to display the frequency of the alternating current. The display unit 320 can display the frequency of the alternating current, making it convenient for the operator to adjust the frequency of the alternating current. For sure, the display unit 320 can also be configured to display information such as product specifications, usage time, or abnormal prompts.

Optionally, a user interface can be displayed on the display unit 320, and the user interface displays the adjustment button 310. The display unit 320 may be a Liquid Crystal Display (LCD) screen.

Optionally, a power supply is further provided on the control module 300, and the power supply is configured to supply power to the units on the control module 300. The power supply may be a battery.

The operation of the guidewire system described above is as follows.

Step 1: an interventional surgical access is opened by puncturing the femoral artery or the radial artery of a patient.

Step 2: the guidewire 200 is placed near a lesion area of the patient along a sheath or a guide catheter under CT angiography.

Step 3: the tail end of the guidewire 200 is fixedly connected to the coil framework 112 through the connecting rod 113.

Step 4: the frequency, amplitude, duty cycle, or duration of the output voltage is set through the control module 300 and output to the driving module 100 through wires.

Step 5: the alternating magnetic field is generated in response to application of the alternating current to the coil framework 112, the reciprocating motion is generated in the coil framework 112 under the action of a fixed magnetic field generated by a magnetic field generating element. The generated mechanical vibration acts on the guidewire 200 and is transmitted to the head end of the guidewire 200, enabling the head end of the guidewire 200 to quickly penetrate the total occlusive lesion.

Step 6: the guidewire 200 is withdrawn along the access, and other relevant therapeutic and diagnostic instruments are used to estimate the treatment effect or proceed with subsequent treatment.

Some other embodiments of the present application also provide a guidewire vibration control method using the guidewire system described in any of the above embodiments. The guidewire vibration control method includes: generating the magnetic field by using the magnetic field generator 120 of the guidewire system, and providing the alternating current to the coil assembly 110 of the guidewire system, enabling the coil assembly 110 to perform axial reciprocating motion in the magnetic field, thereby driving the guidewire 200 of the guidewire system to generate axial vibration.

In the above guidewire vibration control method, when an alternating current is provided to the coil assembly 110, the coil assembly 110 can perform the axial reciprocating motion in the magnetic field generated by the magnetic field generator 120, thereby enabling the coil assembly 110 to drive the guidewire 200 to generate the axial vibration, causing the head end of the guidewire 200 to produce an impact force on the calcified lesion tissue (such as CTO tissue). This impact force can cause fragmentation or atomization of calcification at the point of action on the lesion tissue, allowing for rapid penetration to the lesion tissue, improving effectiveness and efficiency for passing through hard lesions, and alleviating the problem of a long time for CTO treatment surgery. Moreover, the present application uses a means of electromagnetic moving coil to generate vibration in the guidewire 200, which does not have the problem of wear in piezoelectric crystals, thus extending the service life of the guidewire system and reducing costs.

Some other embodiments of the present application also provide a computer-readable storage medium, storing a computer program, which when executed by a processor, implements the steps of the guidewire vibration control method described above.

The technical features of the above-described embodiments can be arbitrarily combined. For the sake of brevity, not all possible combinations of the technical features in the above embodiments have been described. However, as long as these combinations of technical features do not contradict each other, they should all be considered as falling within the scope of this specification.

The above-described embodiments only describe several implementations of the present application. Their descriptions are relatively specific and detailed, but should not be understood as limitations on the protection scope of the present application. It should be pointed out that for those skilled in the art, without departing from the inventive concept of the present application, modifications and improvements can still be made, all of which fall within the protection scope of the present application. Therefore, the protection scope of the present application should be based on the appended claims.

## Claims

1. A guidewire system, **characterized by**, comprising:
a guidewire (200); and
a driving module (100) comprising:
a magnetic field generator (120), configured to generate a magnetic field; and
a coil assembly (110), wherein when an alternating current is provided to the coil assembly (110), the coil assembly (110) is confiuged to perform axial reciprocating motion in the magnetic field and thus drive the guidewire (200) to generate axial vibration.

2. The guidewire system according to claim 1, wherein the coil assembly (110) comprises a coil (111) and a coil frame (112), the coil (111) is disposed on the coil frame (112), and the coil frame (112) is connected to a tail end of the guidewire (200).

3. The guidewire system according to claim 2, wherein an axial guide slot (112a) is provided on the coil frame (112), and a tail end of the magnetic field generator is positioned in the axial guide slot (112a), enabling the coil frame (112) to perform axial reciprocating motion along the magnetic field generator (120), wherein the coil (111) is wound around an outer wall of the coil frame (112).

4. The guidewire system according to claim 3, wherein the coil assembly (110) further comprises a connecting rod (113), a head end of the connecting rod (113) is connected to the tail end of the guidewire (200), and a tail end of the connecting rod (113) is connected to the coil frame (112) after passing through the magnetic field generator (120) and the axial guide slot (112a) in sequence.

5. The guidewire system according to claim 3, wherein the magnetic field generator (120) comprises a large diameter section (121) and a small diameter section (122), the large diameter section (121) and the small diameter section (122) are connected in sequence along a direction from head ends to tail ends of their own, an outer diameter of the large diameter section (121) is greater than that of the small diameter section (122), the outer diameter of the large diameter section (121) is greater than a slot width of the axial guide slot (112a), and the coil frame (112) is configured to perform axial reciprocating motion along the small diameter section (122).

6. The guidewire system according to claim 2, wherein the coil assembly (110) further comprises a magnetic yoke, and the magnetic yoke is disposed on the coil frame (112) and distributed around the coil (111).

7. The guidewire system according to claim 2, wherein the coil assembly (110) further comprises a shock absorber (114), and the shock absorber (114) is disposed between the coil frame (112) and the magnetic field generator (120).

8. The guidewire system according to claim 7, wherein the shock absorber (114) is a spring.

9. The guidewire system according to any one of claims 1 to 8, wherein the driving module (100) further comprises a housing (130), both the coil assembly (110) and the magnetic field generator (120) are disposed inside the housing (130), and a tail end of the guidewire enters the housing (130) to connect with the coil assembly (110).

10. The guidewire system according to any one of claims 1 to 8, wherein outer diameter of a head end of the guidewire (200) gradually decrease in a direction away from the magnetic field generator (120).

11. The guidewire system according to any one of claims 1 to 8, wherein a material of a head end of the guidewire (200) is a thermosensitive material, wherein hardness of the thermosensitive material at a first preset temperature is greater than the hardness of the thermosensitive material at a second preset temperature, and the first preset temperature is greater than the second preset temperature.

12. The guidewire system according to any one of claims 1 to 8, wherein a surface of a head end of the guidewire (200) is covered with a developing layer (210).

13. The guidewire system according to any one of claims 1 to 8, wherein the guidewire system further comprises a control module (300), and the control module (300) is configured to provide the alternating current to the coil assembly (110) and adjust a frequency of the alternating current.

14. The guidewire system according to claim 13, wherein an adjustment button (310) is provided on the control module (300), and the control module (300) adjusts the frequency of the alternating current when it is detected that the adjustment button (310) is pressed.

15. The guidewire system according to claim 13, wherein a display unit (320) is provided on the control module (300), and the display unit (320) is configured to display the frequency of the alternating current.

16. A guidewire vibration control method using the guidewire system according to any one of claims 1 to 15, **characterized by**, comprising:
generating the magnetic field by using the magnetic field generator (120) of the guidewire system, and providing the alternating current to the coil assembly (110) of the guidewire system, enabling the coil assembly (110) to perform axial reciprocating motion in the magnetic field, thereby driving the guidewire (200) of the guidewire system to generate axial vibration.

17. A computer-readable storage medium storing a computer program thereon, **characterized in that**, the computer program, when executed by a processor, implements the steps of the guidewire vibration control method according to claim 16.
